# EUROPÄISCHE PATENTSCHRIFT

(11) **EP 3 181 187 B1**
(45) Veröffentlichungstag und Bekanntmachung des Hinweises auf die Patenterteilung: **19.02.2020**
(21) Anmeldenummer: 16199793.7
(22) Anmeldetag: 21.11.2016
(51) Int. Cl.: A61N 1/05

(54) **IMPLANTIERBARE ELEKTRODE MIT HOHLZYLINDRISCHEM MANTEL**
IMPLANTABLE ELECTRODE WITH HOLLOW CYLINDRICAL SHEATH
ÉLECTRODE IMPLANTABLE COMPRENANT UNE ENVELOPPE CYLINDRIQUE CREUSE

(30) Priorität: 15.12.2015 DE 102015121813
(43) Veröffentlichungstag der Anmeldung: 21.06.2017
(73) Patentinhaber: BIOTRONIK SE & Co. KG, 12359 Berlin (DE)
(72) Erfinder: BIHLER, Eckardt, 8406 Winterthur (CH)
(74) Vertreter: Galander, Marcus

(56) Entgegenhaltungen:
- WO-A1-2006/047178
- WO-A1-2009/099883
- WO-A1-2013/188227
- US-A1- 2014 031 907

## Beschreibung

Die Erfindung betrifft eine implantierbare Elektrode mit einem sich entlang der Längsachse der Elektrode erstreckenden Mantel aus einem elektrisch isolierenden Material, und mit wenigstens einer Verbindungsleitung. Ferner betrifft die Erfindung ein Verfahren zum Herstellen einer implantierbaren Elektrode.

Implantierbare Elektroden und Verfahren zu deren Herstellung sind allgemein bekannt. Sollen die bekannten Elektroden jedoch mindestens eine und womöglich sogar mehrere Verbindungsleitungen zur Verbindung von Kontaktelementen der Elektrode mit einem Gerät aufweisen, so sind bekannte Elektroden jedoch aufwendig und teuer in der Herstellung. So ist die wenigstens eine Verbindungsleitung aufwendig zu ummanteln. Wird zur Bereitstellung der Verbindungsleitung ein Flachbandkabel eingesetzt, so ist dieses nur in einer Vorzugsrichtung flexibel hin und her ist daher schwierig zu implantieren.

Die PCT-Anmeldung WO2009099883A1 beschreibt eine Implantierbare Elektrodenleitung mit einem sich entlang der Längsachse der Elektrodenleitung erstreckenden Mantel aus einem elektrisch isolierenden Material, und mit wenigstens einer Verbindungsleitung, die zumindest teilweise in den Mantel eingebettet und der Mantel hohlzylindrisch ausgeformt ist.

Der Erfindung liegt daher die Aufgabe zugrunde, eine implantierbare Elektrode und ein Verfahren zum Herstellen einer implantierbaren Elektrode bereitzustellen, wobei die implantierbare Elektrode einfach und preiswert hergestellt werden kann und ohne weiteres implantierbar ist.

Für die eingangs genannte Elektrode ist die Aufgabe dadurch gelöst, dass die Verbindungsleitung zumindest teilweise in den Mantel eingebettet und der Mantel hohlzylindrisch ausgeformt ist. Für das eingangs genannte Verfahren ist die Aufgabe dadurch gelöst, dass zumindest eine Verbindungsleitung in einen Mantel der Elektrode eingebettet und der Mantel hohlzylindrisch ausgeformt wird.

Dadurch, dass die Verbindungsleitung in den Mantel eingebettet ist, braucht die Verbindungsleitung nicht mehr separat von einem Isolator umgeben zu sein. Insbesondere wenn die Elektrode mehrere Verbindungsleitungen aufweist, kann der Mantel zur isolierenden Aufnahme der Verbindungsleitungen ausreichen. In einem normalen mehradrigen Kabel separat vorhandene Isolationsschichten zur Isolation der Verbindungsleitungen zueinander sind nicht notwendig. Hierdurch verringert sich nicht nur der Aufwand für die Herstellung, sondern auch der Durchmesser der Elektrode. Durch die hohlzylindrische Ausformung des Mantels hat die Elektrode keine Vorzugsrichtung, in der sie einfacher verformbar ist als in anderen Richtungen. Folglich kann die implantierbare Elektrode einfach durch den Körper des Patienten geschoben und dabei in unterschiedliche Richtungen verformt werden.

Die Elektrode weist zumindest ein Kontaktelement auf, wobei das zumindest eine Kontaktelement durch das Mantelmaterial hindurch elektrisch leitfähig mit der Verbindungsleitung verbunden ist. Die Verbindung zwischen der Verbindungsleitung und dem Kontaktelement ist in Form einer sogenannten Durchkontaktierung. Durch die Kontaktierung der Verbindungsleitung mit dem Kontaktelement durch das Mantelmaterial hindurch, ist eine separate Isolation der Durchkontaktierung nicht notwendig und die Durchkontaktierung verbraucht wenig Platz.

Das zumindest eine Kontaktelement kontaktiert im implantierten Zustand der Elektrode Muskelzellen, beispielsweise des Herzens, oder Nervenenden elektrisch, um die Muskelzellen oder Nervenenden elektrisch stimulieren zu können. Die wenigstens eine Verbindungsleitung kontaktiert das zumindest eine Kontaktelement, insbesondere an einem distalen Ende der Elektrode, und verläuft zu einem gegenüberliegenden Ende der Elektrode, wo es signalübertragend mit einem medizinischen Gerät verbindbar oder sogar verbunden sein kann.

Das zumindest eine Kontaktelement kann sich mindestens teilweise oder sogar vollständig um die Längsachse der Elektrode herum erstrecken, um bei der Implantation der Elektrode eine Kontaktierung der Muskelzellen oder Nervenenden zu vereinfachen.

Die Elektrode kann mehrere zumindest teilweise oder vollständig in den Mantel eingebettete Verbindungsleitungen aufweisen, wobei die Verbindungsleitungen in einer sich um die Längsachse herum erstreckenden Umfangsrichtung des Mantels beabstandet zueinander angeordnet sind. Dadurch, dass die Verbindungsleitung in der Umfangsrichtung beabstandet zueinander angeordnet sind, können sie einfach und kollisionsfrei durch den Mantel geführt sein. Um die Eigenschaft der Elektrode, dass diese ohne Vorzugsrichtung verformbar und beispielsweise biegbar ist, weiter zu verbessern, können die Verbindungsleitungen in der Umfangsrichtung gleichverteilt um die Längsachse herum angeordnet sein.

Um mehrere Muskelzellbereiche oder Nervenenden kontaktieren zu können, kann die Elektrode mehrere Kontaktelemente aufweisen. Jedes der Kontaktelemente kann mit einer der Verbindungsleitungen elektrisch leitfähig verbunden sein. Insbesondere kann jedes der Kontaktelemente durch das Mantelmaterial hindurch elektrisch leitfähig mit zumindest einer der Verbindungsleitungen verbunden sein.

Um zu ermöglichen, dass mehrere oder alle der Kontaktelemente sich zumindest teilweise oder sogar vollständig um die Längsachse der Elektrode herum erstrecken und somit einfach in Kontakt mit Muskelzellen oder Nervenenden gebracht werden können, können die Kontaktelemente entlang der Längsachse beabstandet zueinander angeordnet sein.

Die Elektrode kann einen Kern aufweisen, an dem der Mantel anliegt. Vorzugsweise ist der Kern zylindrisch oder hohlzylindrisch und beispielsweise schlauchförmig ausgebildet.

Der Kern kann verhindern, dass der Mantel bei einem womöglich zu starkem Biegen, also bei einem zu kleinen Biegeradius, einknickt und entstehende Knickkanten oder -ecken die Implantation der Elektrode erschweren.

Der Mantel kann stoffschlüssig und zum Beispiel durch Verschmelzen mit dem Kern verbunden sein, wodurch die Verbindung zwischen Mantel und Kern ohne weitere Hilfsmittel, beispielsweise Kleber oder Nähte, ausgebildet sein kann.

Der Kern kann mit einem sich entlang der Längsachse der Elektrode durchgängig durch den Kern erstreckenden Lumen ausgebildet sein, damit die Elektrode beispielsweise mit Hilfe eines Führungsdrahtes, der die Elektrode bei der Implantation führt, implantierbar ist. Ist die Elektrode ohne Kern ausgebildet, kann der hohlzylindrische Mantel das Lumen bereitstellen.

Das Material des Mantels kann ein thermoplastisches Polymer, beispielsweise ein Flüssigkristall-Polymer sein. Der Kern kann beispielsweise aus Glas oder ebenfalls aus einem Polymer, zum Beispiel aus einem Flüssigkristall-Polymer, gefertigt sein und sogar daraus bestehen. Die Verbindungsleitung besteht zum Beispiel aus Gold oder aus einer Goldlegierung. Das zumindest eine Kontaktelement kann aus Gold, aus einer Goldlegierung oder zum Beispiel aus einer Platin-Iridium-Legierung gefertigt sein.

Der Mantel ist vorzugsweise zumindest nahezu ringförmig ausgestaltet und kann in und entgegen der Umfangsrichtung Enden aufweisen, die einander in der Umfangsrichtung gegenüber stehen und gemeinsam parallel zur Längsachse verlaufen.

Die zumindest eine Verbindungsleitung oder ausgewählte sowie alle der mehreren Verbindungsleitungen können parallel zur Längsachse ausgerichtet sein. Alternativ kann die wenigstens eine Verbindungsleitung oder können die mehreren Verbindungsleitungen spiral-oder helixförmig, also beispielsweise gewendelt, um die Längsachse herum verlaufen. Außerdem besteht die Möglichkeit, dass die wenigstens eine Verbindungsleitung beziehungsweise ausgewählte oder alle der mehreren Verbindungsleitungen mäandernd, also zumindest abschnittsweise quer zur Längsachse der Elektrode, im Mantelmaterial verlaufend angeordnet sind. Darüber hinaus können die Verbindungsleitungen miteinander verflochten angeordnet sein, wobei zwischen den Verbindungsleitungen isolierendes Mantelmaterial verbleiben kann. Hierdurch kann nicht nur die Verformbarkeit in verschiedene Richtung verbessert werden. Ist das Mantelmaterial entlang der Längsachse elastisch, kann zumindest der Verbindungsabschnitt der Elektrode aufgrund der Form der Verbindungsleitungen entlang der Längsachse dehnbar sein.

Gemäß der Erfindung ist der Mantel flächig ausgebildet und die zumindest eine Verbindungsleitung in den flächigen Mantel eingebettet, wobei der flächige Mantel mit der zumindest einen eingebetteten Verbindungsleitung in die hohlzylindrische Form gebracht ist. Der Mantel ist als ein Flachbandkabel ausgebildet, wobei die zumindest eine Verbindungsleitung zwischen zwei elektrisch isolierende thermoplastische Polymerfolien, zum Beispiel aus einem Flüssigkristall-Polymer, angeordnet ist. Eine der Folien ist auf einer ihrer Seiten mit der zumindest einen Verbindungsleitung versehen. Auf der der Verbindungsleitung gegenüberliegenden Seite dieser Folie ist die Folie mit dem wenigstens einen Kontaktelement versehen. Die Verbindungsleitung und/oder das Kontaktelement können additiv oder subtraktiv auf die Folie aufgebracht werden. Um die Verbindungsleitung und das Kontaktelement miteinander in Verbindung bringen zu können, ist die Durchkontaktierung als ein sogenanntes metallisiertes Via ausgebildet. Ein Via ist eine die beiden Seiten der Folie miteinander verbindende metallisierte Öffnung in der Folie.

Die Folie kann mit dem Kontaktelement und der Verbindungsleitung um den Kern herum gelegt werden. Alternativ oder zusätzlich kann eine weitere Folie auf die Seite der Folie, die die Verbindungsleitung aufweist, aufgelegt werden. Insbesondere wenn die Folien thermoplastische Polymerfolien sind, können die beiden Folien thermisch dauerhaft miteinander verbunden werden. Mit der ohne das Kontaktelement ausgeformten Folie kann der Mantel dann um den Kern herum gelegt werden.

Um den Mantel mit dem Kern verbinden zu können, kann der Mantel zunächst um den Kern herumgelegt werden, wobei der Mantel sich insbesondere entlang der Umfangsrichtung an den Kern anschmiegen und diesen zumindest teilweise oder sogar vollständig umgeben kann.

Vorzugsweise entspricht eine Breite des flächigen Mantels im Wesentlichen dem Außenumfang des Kerns, sodass der Mantel den Kern im Wesentlich vollständig umgeben kann, ohne dass eine Kante des flächigen Mantels die gegenüberliegende Kante des flächigen Mantels überlappt.

Um den Mantel und den Kern einfach aneinander befestigen zu können, können der Mantel und der Kern stoffschlüssig miteinander verbunden werden. Insbesondere wenn sowohl das Mantelmaterial als auch der Kern aus einem thermoplastischen Polymermaterial gefertigt sind, können der Mantel und der Kern unter Temperatur- und Druckeinwirkung einfach aneinander befestigt und zum Beispiel verschmolzen werden. Zum Beispiel kann der Mantel von einer Form gegen den Kern gedrückt werden, wobei die Form vorzugsweise nicht am Mantel haftet und beispielsweise Teflon- oder Keramikoberflächen aufweist, die den Mantel bei der Herstellung der Elektrode kontaktieren. Um den Mantel an dem Kern befestigen zu können, können Mantel und Kern auf bis zu 350°C und beispielsweise auf 200°C erwärmt werden. Diese Temperaturen können für bis zu 20 Minuten und beispielsweise fürs wenigstens 1 Sekunde aufrechterhalten werden. Das Material des Mantels und Kerns können bei einer derartigen Temperaturbehandlung miteinander verschmelzen und sich so nahtlos oder zumindest nahezu nahtlos stoffschlüssig miteinander verbinden.

Beispielsweise kann als Flüssigkristall-Polymer Polyurethan oder ein anderes Polymer verwendet werden. Weist der Kern Glas- oder Karbonfasern auf, so können diese Fasern mit thermoplastischem Polymermaterial ummantelt sein, um den Mantel mit dem Kern einfach stoffschlüssig verbinden zu können.

Der Durchmesser der Elektrode kann zwischen 200 µm und 700 µm und beispielsweise 250 µm, 400 µm oder 500 µm betragen. Ein Abstand der zumindest einen Verbindungsleitung zur Längsachse kann zwischen 50 µm und 250 µm und beispielsweise 90 µm, 165 µm oder 215 µm betragen. Sind mehrere Verbindungsleitungen vorgesehen, können diese auf einem die Längsachse umgebenden Ring angeordnet sein, wobei der Ring einen Durchmesser zwischen 100 µm und 500 µm und beispielsweise 180 µm, 330 µm oder 430 µm aufweisen kann. Der Durchmesser des Kerns kann zwischen 100 µm und 400 µm und beispielsweise 110 µm, 260 µm oder 360 µm betragen. Bei einem Abstand von 25 µm zwischen den Verbindungsleitungen und in der Umfangsrichtung kann die Verbindungsleitung bis zu 11, bis zu 21 oder sogar bis zu 30 und beispielsweise 27 Verbindungsleitungen aufweisen. Bei einem Abstand von 15 µm zwischen den Verbindungsleitungen und in der Umfangsrichtung kann die Elektrode bis zu 19, bis zu 35 oder sogar bis zu 50 und beispielsweise 45 Verbindungsleitungen aufweisen. Jede der Verbindungsleitungen kann mit einem separaten Kontaktelemente elektrisch leitfähig verbunden sein. Es kann jedoch ausreichen, dass die Elektrode zwei oder drei Verbindungsleitungen und Kontaktelemente aufweist, beispielsweise wenn die Elektrode zum Verbinden eines Herzschrittmachers oder Defibrillator mit dem Herzen ausgebildet ist.

Im Folgenden ist die Erfindung beispielhaft anhand von Ausführungsformen mit Bezug auf die Zeichnungen erläutert. Die unterschiedlichen Merkmale der Ausführungsformen können dabei unabhängig voneinander kombiniert werden, wie es bei den vorteilhaften Ausgestaltungen bereits dargelegt wurde. Es zeigen:
- Fig. 1: eine schematische Darstellung eines Ausführungsbeispiels der erfin-dungsgemäßen implantierbaren Elektrode in einer perspektivischen An-sicht,
- Fig. 2: eine schematische Schnittdarstellung eines weiteren Ausführungsbei-spiels der erfindungsgemäßen implantierbaren Elektrode,
- Fig. 3 bis 5: schematische Darstellungen eines Ausführungsbeispiels eines Mantels der erfindungsgemäßen Elektrode, wobei der Mantel flächig als ein Halbprodukt dargestellt ist,
- Fig. 6: eine schematische Schnittdarstellung eines Ausführungsbeispiels des flächigen Mantels mit einem Kern,
- Fig. 7: eine schematische Schnittdarstellung des um den Kern herum gelegten Mantels des Ausführungsbeispiels der Figur 6,
- Fig. 8: eine schematische Schnittdarstellung des Ausführungsbeispiels der Figur 7 nach einer thermischen Behandlung, und
- Fig. 9: eine schematische Darstellung eines Ausführungsbeispiels des erfindungsgemäßen Verfahrens zum Herstellen der implantierbaren Elektrode.

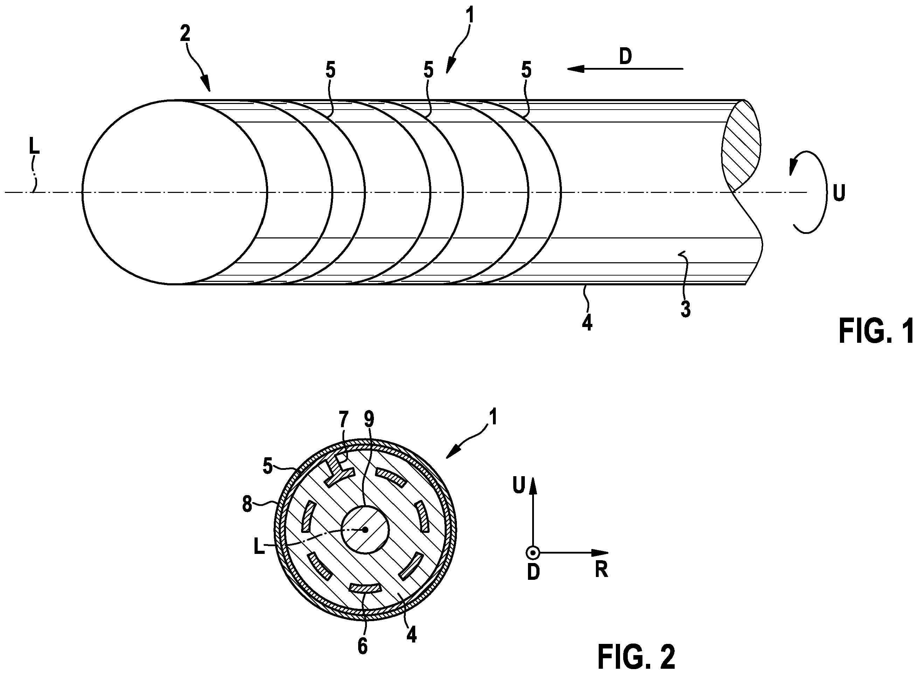

Zunächst sind Aufbau und Funktion einer erfindungsgemäßen implantierbaren Elektrode mit Bezug auf das Ausführungsbeispiel der Figur 1 beschrieben.

Figur 1 zeigt die implantierbare Elektrode 1 schematisch in einer perspektivischen Ansicht. Insbesondere zeigt die Figur 1 ein distales Ende 2 der Elektrode 1. Das distale Ende 2 ist ausgebildet, Muskelzellen oder Nervenenden elektrisch zu kontaktieren, um diese stimulieren zu können. Alternativ kann das Ende 2 auch ein dem distalen Ende der Elektrode 1 gegenüberliegendes Anschlussende der Elektrode 1 sein und zum Anschluss der Elektrode 1 an ein medizinisches Gerät, beispielsweise ein Herzschrittmacher oder ein Defibrillator, sein. Die implantierbare Elektrode 1 weist eine Längsachse L auf, die sich zentral durch die Elektrode 1 erstreckt. Die implantierbare Elektrode 1 ist in allen quer zur Längsachse L weisenden Richtungen verformbar und insbesondere bieg- oder schwenkbar.

Die Elektrode 1 ist mit einer Außenseite 3 eines Mantels 4 der Elektrode 1 dargestellt. Auf dem Mantel 4 und von außerhalb der Elektrode 1 zugänglich, weist die Elektrode 1 des Ausführungsbeispiels der Figur 1 drei Kontaktelemente 5 auf. Die implantierbare Elektrode 1 kann auch mehr oder weniger als drei Kontaktelemente 5 aufweisen. Beispielsweise kann die Elektrode 1 ein Kontaktelement 5 oder mehr als ein Kontaktelement 5 und beispielsweise zwei, vier, fünf, bis zu 11, 19, 21, 35, 27, 30, 45 oder sogar bis zu 50 Kontaktelemente 5 aufweisen.

Die Außenseite 3 des Mantels 4 verläuft parallel zur Längsachse L und erstreckt sich in einer Umfangsrichtung U der Elektrode 1 um die Längsachse L herum. Die Kontaktelemente 5 erstrecken sich in der Umfangsrichtung U zumindest teilweise oder sogar vollständig um die Längsachse L herum, sodass sie aus mehreren oder sogar allen auf die Längsachse L zu weisenden Richtungen kontaktierbar sind. Die Kontaktelemente 5 können also als Kontaktringe bezeichnet werden.

In einer parallel zur Längsachse L verlaufenden und auf das distale Ende 2 zu weisenden Längsrichtung D der Elektrode 1 sind die Kontaktelemente 5 beabstandet zueinander angeordnet. Somit ist gewährleistet, dass die Kontaktelemente 5 einander nicht ungewollt elektrisch kontaktieren, sondern vom Mantel 4 voneinander elektrisch isoliert sind.

Figur 2 zeigt ein weiteres Ausführungsbeispiel der implantierbaren Elektrode 1 in einer schematischen Schnittansicht. Für Elemente, die in Form und/oder Funktion Elementen des vorherigen Ausführungsbeispiels entsprechen, sind dieselben Bezugszeichen verwendet. Der Kürze halber ist im Folgenden lediglich auf die Unterschiede zum vorherigen Ausführungsbeispiel eingegangen.

Die Schnittebene verläuft quer zur Längsachse L und durch eines der Anschlusskontaktelemente 5 der Elektrode 1. Die Elektrode 1 des Ausführungsbeispiels der Figur 2 weist mehrere Verbindungsleitungen 6 und beispielhaft sieben Verbindungsleitungen 6 auf. Jede der Verbindungsleitungen 6 kann mit einem anderen Anschlusskontaktelement 5 der Elektrode 1 elektrisch leitfähig verbunden sein. Im Ausführungsbeispiel der Figur 2 verbindet eine Durchkontaktierung 7, die auch als Via bezeichnet sein kann, eine der Verbindungsleitungen 6 mit dem Kontaktelement 5, durch die sich die Schnittebene erstreckt.

Die Verbindungsleitungen 6 sind in den Mantel 4 eingebettet. Die Durchkontaktierung 7 verläuft von dem dargestellten Kontaktelement 5 zu einer der Verbindungsleitungen 6 und erstreckt sich dabei ebenfalls durch das Material des Mantels 4. Insbesondere kann sich die Durchkontaktierung 7 in einer quer zur Längsachse L verlaufenden radialen Richtung R zwischen dem dargestellten Kontaktelement 5 und dem mit diesem Kontaktelement 5 elektrisch leitfähig verbundenen Verbindungselement 6 erstrecken. Jede der Verbindungsleitungen 6 kann mit Hilfe einer anderen Durchkontaktierung elektrisch leitfähig 7 mit jeweils einem der Kontaktelemente 5 verbunden sein.

Auf seiner von der Längsachse L wegweisenden Außenseite kann das Kontaktelement 5 mit einer metallischen Schicht 8 versehen sein, wobei die metallische Schicht beispielsweise eine Platin-Iridium-Legierung ist.

Der Mantel 4 kann sich um einen zylindrischen Hohlraum herum erstrecken, der beispielsweise als ein Lumen zur Aufnahme eines Führungsdrahtes ausgebildet ist. Im Ausführungsbeispiel der Figur 2 ist die Elektrode 1 jedoch mit einem Kern 9 dargestellt, um den sich der Mantel 4 vollständig herum erstreckt. Der Kern 9 kann selbst als ein Vollzylinder oder hohlzylindrisch, etwa schlauchförmig, ausgebildet sein. Der Mantel 4 kann am Kern 9 befestigt und beispielsweise stoffschlüssig mit dem Kern 9 verbunden sein.

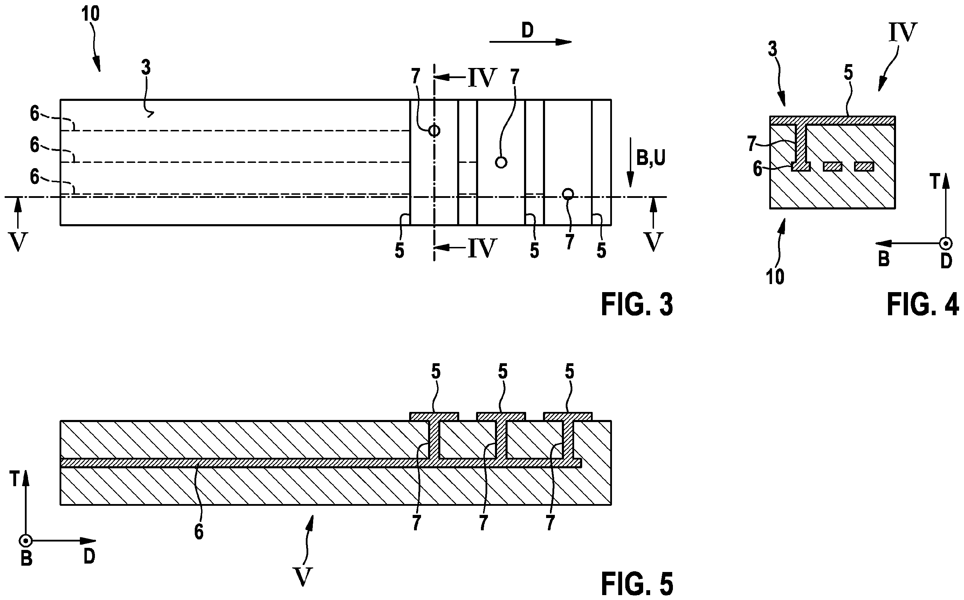

Figuren 3 bis 5 zeigen einen flächigen Mantel als ein Halbprodukt eines weiteren Ausführungsbeispiels der implantierbaren Elektrode 1 schematisch in einer Aufsicht und in geschnittenen Ansichten. Für Elemente, die in Funktion und/oder Aufbau Elementen der Ausführungsbeispiele der Figuren 1 und 2 entsprechen, sind dieselben Bezugszeichen verwendet. Der Kürze halber ist im Folgenden lediglich auf die Unterschiede zu den Ausführungsbeispielen der Figuren 1 und 2 eingegangen.

Figur 3 zeigt einen flächigen Mantel 10 als Halbfabrikat des Mantels 4 der Elektrode 1. Die Außenseite 3 des flächigen Mantels 10 entspricht der Außenseite 3 des Mantels 4 der Elektrode 1. Auf der Außenseite 3 des flächigen Mantels 10 sind drei streifenförmige Kontaktelemente 5 angeordnet, die in einer Längsrichtung D des flächigen Mantels 10, die der Längsrichtung D der Elektrode 1 entspricht, hintereinander und beabstandet voneinander angeordnet sind. In einer Breitenrichtung B, die im montierten Zustand des flächigen Mantels 10 der Umfangsrichtung U entspricht, können die Kontaktelemente 5 die Außenseite 3 vollständig bedecken.

Jedes der Kontaktelemente 5 kann mit einer anderen Verbindungsleitung 6 elektrisch leitfähig verbunden sein. Somit kann der flächige Mantel 10 beispielsweise drei Verbindungsleitungen 6 aufweisen. Jede der Verbindungsleitungen 6 kann mit Hilfe einer anderen Durchkontaktierung 7 mit jeweils einem der Kontaktelemente 5 elektrisch leitfähig verbunden sein.

Im Ausführungsbeispiel der Figur 3 erstrecken sich die Verbindungsleitungen 6 nur bis zu der Durchkontaktierung 7, welche die entsprechende Verbindungsleitung 6 mit dem jeweiligen Kontaktelement 5 elektrisch leitfähig verbindet. Alternativ können sich die Verbindungsleitungen 6 jedoch auch in der Längsrichtung D bis hinter diese Durchkontaktierung 7 und sich sogar vollständig durch den flächigen Mantel 10 erstrecken. Eine ungewollte Kontaktierung einer der Verbindungsleitungen 6 mit einem anderen Kontaktelement 5 ist dadurch verhindert, dass das Kontaktelement 5 auf der Außenseite 3 und die Verbindungsleitungen 6 in einer quer zur Längsrichtung D und zur Breitenrichtung B weisenden Dickenrichtung T beabstandet von der Außenseite 3 angeordnet sind.

Figur 4 zeigt den flächigen Mantel 10 des Ausführungsbeispiels der Figur 3 schematisch in einer Schnittansicht IV, die sich quer zur Längsrichtung D durch eines der Kontaktelemente 5 erstreckt. Die Längsrichtung D weist aus der Zeichenebene heraus. In der Breitenrichtung B sind die Verbindungsleitungen 6 nebeneinander und beabstandet zueinander angeordnet. In der Dickenrichtung T sind die Verbindungsleitungen 6 beabstandet zum Kontaktelement 5 vorgesehen. Die Durchkontaktierung 7, die das dargestellte Kontaktelement 5 mit einer der Verbindungsleitungen 6 elektrisch leitfähig verbindet, verläuft in der Dickenrichtung T von der Verbindungsleitung 6 zum Kontaktelement 5.

Die Verbindungsleitungen 6 können auf einer der Außenseite 3 gegenüberliegende Innenseite des flächigen Mantels 10 angeordnet sein. Im Ausführungsbeispiel der Figur 4 sind die Verbindungsleitungen 6 jedoch so in den flächigen Mantel 10 eingebettet, dass die Verbindungsleitungen 6 quer zur Längsrichtung D nur über eines der Kontaktelemente 5 und die jeweilige Durchkontaktierung 7 kontaktierbar sind.

Figur 5 zeigt den flächigen Mantel 10 des Ausführungsbeispiels der Figur 3 in einer Schnittansicht V, wobei die Schnittebene durch eine der Verbindungsleitungen 6, also parallel zur Längsrichtung D und zur Dickenrichtung T, verläuft.

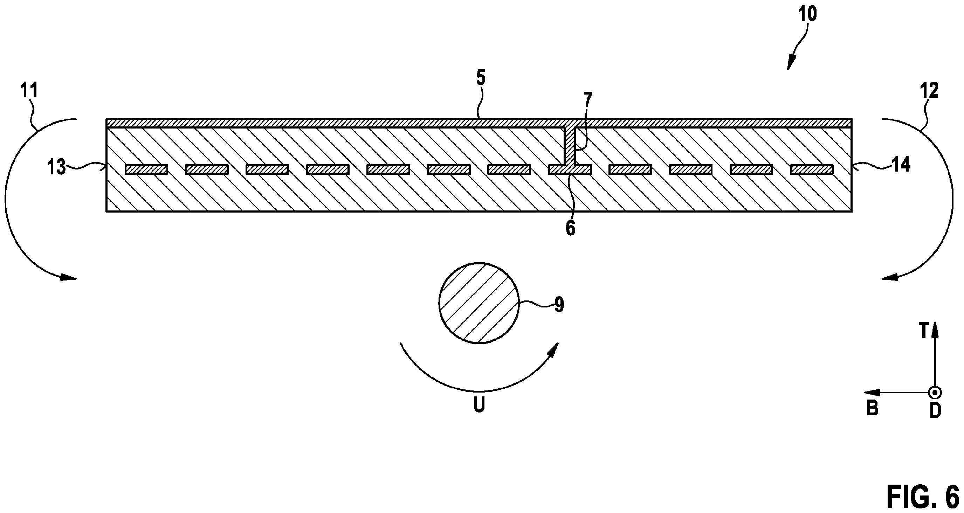

Figur 6 zeigt ein weiteres Ausführungsbeispiels des flächigen Mantels 10 in einer Schnittansicht. Für Elemente, die in Funktion und/oder Aufbau Elementen der bisherigen Ausführungsbeispiele entsprechen, sind dieselben Bezugszeichen verwendet. Der Kürze halber ist im Folgenden lediglich auf die Unterschiede zu den bisherigen Ausführungsbeispielen eingegangen.

Die Schnittebene verläuft vergleichbar zur Figur 4 parallel zur Breitenrichtung B und zur Dickenrichtung T durch ein Kontaktelement 5 hindurch. In der Schnittansicht der Figur 6 verbindet eine Durchkontaktierung 7 eine der Verbindungsleitungen 6 des flächigen Mantels 10 mit einem Kontaktelement 5. Der flächige Mantel 10 weist mehrere und beispielsweise zehn Verbindungsleitungen 6 auf, von denen jedoch nur eine in der Figur 6 sichtbar durch die Durchkontaktierung 7 mit dem Kontaktelement 5 verbunden ist. Je Verbindungsleitung 6 kann der flächige Mantel 10 jedoch ein Kontaktelement 5 aufweisen, wobei jedes der Kontaktelemente 5 durch eine Durchkontaktierung 7 mit einer der Verbindungsleitungen 6 elektrisch leitfähig verbunden sein kann. In und/oder entgegen der Längsrichtung D sind jedoch nicht nur die Kontaktelemente 5, sondern auch die Durchkontaktierungen 7, welche die nicht dargestellten Kontaktelemente 5 mit einer der Verbindungsleitungen 6 elektrisch verbinden, vor beziehungsweise hinter der Schnittebene dargestellt, sodass diese nicht sichtbar sind.

Wie durch die beiden gekrümmten Pfeile 11, 12 dargestellt, kann der flächige Mantel 10 so um den Kern 9 herum gelegt werden, dass sich der Mantel 10 in der Umfangsrichtung U um den Kern 9 herumlegt und den Mantel 4 ausbildet. Alternativ zum Kern 9 kann der flächige Mantel 10 um ein Anlegewerkzeug herum gelegt werden, das später entfernt wird, um die Elektrode 1 mit einem sich entlang der Längsachse L durch die Elektrode 1 erstreckenden Lumen zu versehen. Auch der Kern 9 kann ein Lumen aufweisen und folglich hohlzylindrisch oder schlauchförmig ausgebildet sein.

Der flächige Mantel 10 kann zumindest teilweise oder sogar vollständig den Kern 9 umschließen, sodass sich dessen parallel zur Längsrichtung D erstreckenden Längsseiten 13, 14 kontaktieren, wenn der flächige Mantel 10 um den Kern 9 herum gelegt wurde. Alternativ kann zwischen den Längsseiten 13, 14 eine Lücke verbleiben, um bei womöglich vorhandenen Breitentoleranzen des flächigen Mantels 10 zu verhindern, dass die Längsseiten 13, 14 einander überlappen.

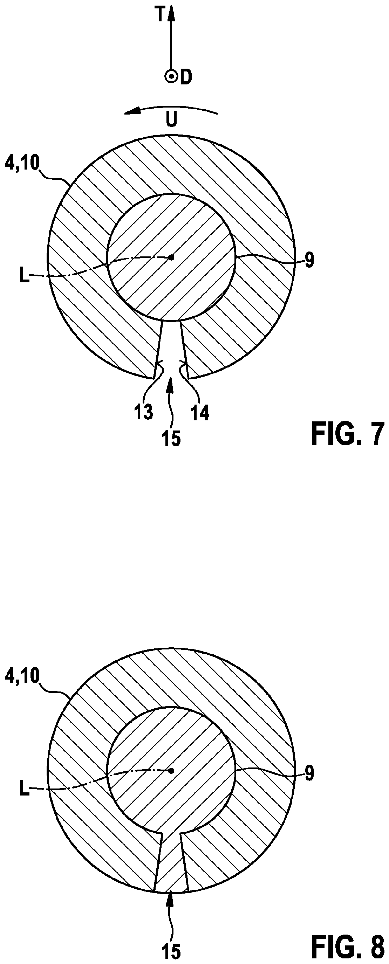

Figuren 7 und 8 zeigen den flächigen Mantel 10 und den Kern 9 beispielsweise des Ausführungsbeispiels der Figur 6 schematisch in derselben Schnittansicht, wobei der flächige Mantel 10 in der Figur 7 um den Kern 9 herum gelegt ist. Zwischen den Längsseiten 13, 14 verbleibt eine Lücke 15, sodass die Längsseiten 13, 14 in der Umfangsrichtung U beabstandet zueinander angeordnet sind. Um den Mantel 10 mit dem Kern 9 fest zu verbinden, können der Mantel 10 und der Kern 9 zumindest teilweise miteinander verschmolzen werden.

Figur 8 zeigt den mit dem Kern 9 verschmolzenen und den Mantel 4 ausbildenden Mantel 10. Haben die Längsseiten 13, 14 beim Verschmelzen des Kerns 9 mit dem Mantel 10 einander kontaktiert, so können auch die Längsseiten 13, 14 miteinander verschmolzen sein. Im Ausführungsbeispiel der Figur 8 verbleibt jedoch die Lücke 15 zwischen den Längsseiten 13, 14. Nach dem Verschmelzen des Kerns 9 mit dem flächigen Mantel 10 kann die Lücke 15 mit erstarrtem Material des Kerns 9 und/oder erstarrtem Material des Mantels 10 gefüllt sein.

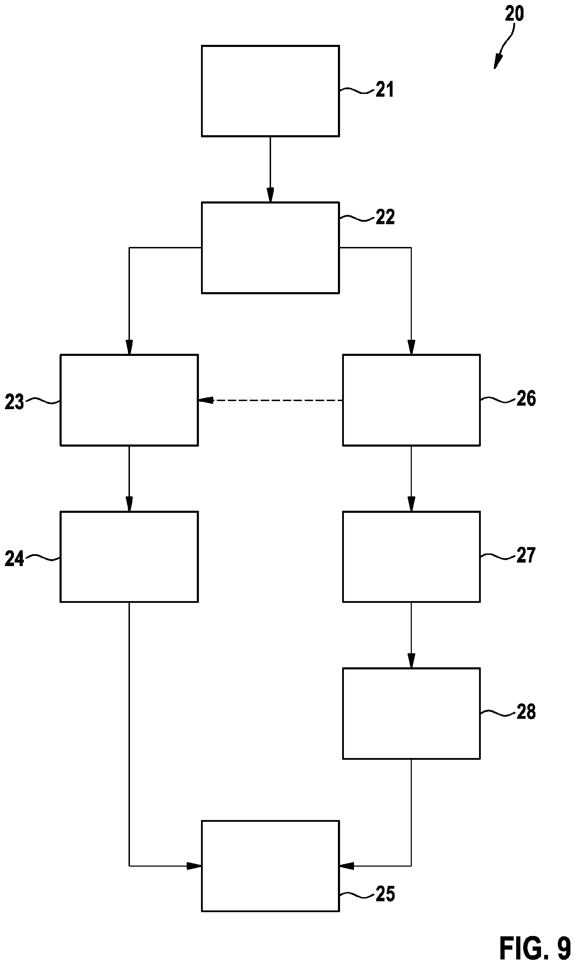

Figur 9 zeigt ein erstes Ausführungsbeispiel des erfindungsgemäßen Verfahrens zum Herstellen einer implantierbaren Elektrode, beispielsweise der implantierbaren Elektrode eines der vorherigen Ausführungsbeispiele. Für Elemente der bisherigen Ausführungsbeispiele, die im Folgenden zur Erläuterung des Verfahrens verwendet sind, sind der Einfachheit halber dieselben Bezugszeichen wie in der bisherigen Beschreibung verwendet.

Das Verfahren 20 startet mit einem ersten Verfahrensschritt 21. Im ersten Verfahrensschnitt 21 kann beispielsweise eine den flächigen Mantel 10 zumindest teilweise ausbildende Folie bereitgestellt werden. Um zumindest eine Verbindungsleitung 6 mit wenigstens einem Kontaktelement 5 elektrisch leitfähig verbinden zu können, kann im Verfahrensschritt 21 die Folie an einer vorbestimmten Position gelocht werden. Beispielsweise kann die Folie mechanisch, etwa mit einem Stanzwerkzeug, mithilfe eines Lasers oder einer Lithografievorrichtung gelocht werden.

Auf den Verfahrensschritt 21 folgt der Verfahrensschritt 22, in dem das Kontaktelement 5, die Verbindungsleitung 6 und die das Kontaktelement 5 und die Verbindungsleitung 6 verbindende Durchkontaktierung 7 an der Folie ausgebildet werden. Beispielsweise kann zunächst das Kontaktelement 5 oder die Verbindungsleitung 6 additiv oder subtraktiv auf einer Seite der Folie vorgesehen werden. Im Anschluss daran kann die Durchkontaktierung in dem ausgebildeten Loch ausgeformt werden. Im Anschluss daran kann das bisher nicht ausgebildete Kontaktelement 5 oder die bisher nicht ausgebildete Verbindungsleitung 6 auf der anderen Seite der Folie ausgeformt werden. Das Kontaktelement 5 wird auf einer Seite und die Verbindungsleitung 6 auf einer dem Kontaktelement 5 gegenüberliegenden Seite der Folie ausgebildet. Das Loch verbindet die beiden Seiten miteinander.

Wird die implantierbare Elektrode 1 mit einem Kern 9 gefertigt, so kann auf den Verfahrensschritt 22 der Verfahrensschritt 23 folgen, in dem der flächige Mantel 10, also die mit dem Kontaktelement 5, der Verbindungsleitung 6 und der Durchkontaktierung 7 versehene Folie, um den Kern 9 herum gelegt wird. Auf den Verfahrensschritt 23 kann der Verfahrensschritt 24 folgen, in dem der Mantel 10 am Kern 9 befestigt und der Mantel 10 hierzu beispielsweise mit dem Kern 9 verschmolzen werden kann. Die Verbindungsleitung 6 kann dabei zumindest teilweise in die aufschmelzende Folie eingebettet werden. Im dann folgenden Verfahrensschritt 25 endet das Verfahren 20.

Alternativ kann auf den Verfahrensschritt 22 der Verfahrensschritt 26 folgen. Im Verfahrensschritt 26 wird auf die Seite der Folie, auf der die Verbindungsleitung 6 angeordnet ist, eine weitere Folie aufgebracht. Die weitere Folie kann eine Innenseite des Mantels 4 ausbilden, sodass die Verbindungsleitung 6 nicht nur teilweise in die eine Folie, sondern vollständig zwischen den Folien in den Mantel 4 eingebettet ist.

Auf den Verfahrensschritt 26 können die Verfahrensschritte 23 bis 25 folgen. Alternativ kann auf den Verfahrensschritt 26 der Verfahrensschritt 27 folgen, in dem der durch die beiden Folien gebildete flächige Mantel 10 hohlzylindrisch gelegt wird. Beispielsweise kann der Mantel 10 um ein Anlegewerkzeug herum gelegt werden, das später aus dem Mantel 4 entfernt wird. Im auf den Verfahrensschritt 27 folgenden Verfahrensschritt 28 können die Längsseiten 13, 14 des hohlzylindrisch gelegten flächigen Mantels 10 aneinander befestigt und beispielsweise miteinander verschmolzen werden, um den hohlzylindrischen Mantel 4 auszuformen. Auf den Verfahrensschritt 28 folgt wieder der Verfahrensschritt 25, in dem das Verfahren endet.

### Bezugszeichenliste

- 1: implantierbare Elektrode
- 2: distales Ende
- 3: Außenseite
- 4: Mantel
- 5: Kontaktelement
- 6: Verbindungsleitung
- 7: Durchkontaktierung
- 8: metallische Schicht
- 9: Kern
- 10: flächiger Mantel (Halbfabrikat)
- 11, 12: Pfeil
- 13, 14: Längsseiten des flächigen Mantels
- 15: Lücke zwischen Längsseiten
- 20: Verfahren
- 21: Folie bereitstellen und lochen
- 22: Kontaktelement, Verbindungselement und Durchkontaktierung ausformen
- 23: flächigen Mantel um Kern herum legen
- 24: flächigen Mantel am Kern befestigen
- 25: Ende
- 26: weitere Folie aufbringen
- 27: flächigen Mantel hohlzylindrisch legen
- 28: Längsseiten aneinander befestigen
- B: Breitenrichtung
- D: Längsrichtung
- L: Längsachse
- R: radiale Richtung
- T: Dickenrichtung
- U: Umfangsrichtung

## Patentansprüche

1. Implantierbare Elektrode (1) mit einem sich entlang der Längsachse (L) der Elektrode (1) erstreckenden Mantel (4) aus einem elektrisch isolierenden Material, und mit wenigstens einer Verbindungsleitung (6), wobei die Verbindungsleitung (6) zumindest teilweise in den Mantel (4) eingebettet und der Mantel (4) hohlzylindrisch ausgeformt ist und mit zumindest einem Kontaktelement (5) zur elektrischen Kontaktierung von Muskelzellen oder Nervenenden, wobei das zumindest eine Kontaktelement (5) durch den Mantel (4) hindurch elektrisch leitfähig mit der Verbindungsleitung (6) verbunden ist
**dadurch gekennzeichnet, dass**
die zumindest eine Verbindungsleitung (6) zwischen zwei elektrisch isolierenden und thermisch dauerhaft miteinander verbundenen thermoplastischen Polymerfolien angeordnet ist, wobei die außen (3) liegende Folie
- auf ihrer Innenseite mit der zumindest einen Verbindungsleitung (6) versehen ist und
- auf ihrer Außenseite (3), die der Verbindungsleitung (6) gegenüberliegende Seite dieser Folie, mit dem wenigstens einen Kontaktelement (5) versehen ist,
wobei zur Verbindung der Verbindungsleitung (6) mit dem Kontaktelement (5) eine Durchkontaktierung (7) in Form einer die beiden Seiten der Folie miteinander verbindende metallisierte Öffnung in der Folie vorgesehen ist.

2. Implantierbare Elektrode nach Anspruch 1, **dadurch gekennzeichnet, dass** Die zumindest eine Verbindungsleitung (6) oder ausgewählte Verbindungsleitungen (6) oder alle der mehreren Verbindungsleitungen (6) parallel zur Längsachse (L) der Elektrode (1) ausgerichtet sind.

3. Implantierbare Elektrode nach Anspruch 1 oder 2, **dadurch gekennzeichnet, dass** sich das zumindest eine Kontaktelement (5) zumindest teilweise um die Längsachse (L) herum erstreckt.

4. Implantierbare Elektrode nach einem der Ansprüche 1 bis 3, **dadurch gekennzeichnet, dass** die Elektrode (1) mehrere zumindest teilweise in den Mantel (4) eingebettete Verbindungsleitungen (6) aufweist, wobei die Verbindungsleitungen (6) in einer sich um die Längsachse (L) herum erstreckenden Umfangsrichtung (U) der Elektrode (1) beabstandet zueinander angeordnet sind.

5. Implantierbare Elektrode nach Anspruch 3 oder 4, **dadurch gekennzeichnet, dass** die Elektrode (1) mehrere Kontaktelemente (5) aufweist.

6. Implantierbare Elektrode nach Anspruch 5, **dadurch gekennzeichnet, dass** die Kontaktelemente (5) entlang der Längsachse (L) beabstandet zueinander angeordnet sind.

7. Implantierbare Elektrode nach einem der Ansprüche 1 bis 6, **dadurch gekennzeichnet, dass** die Elektrode (1) einen Kern (9) aufweist, an dem der Mantel (4) anliegt.

8. Implantierbare Elektrode nach Anspruch 7, **dadurch gekennzeichnet, dass** der Mantel (4) stoffschlüssig mit dem Kern (9) verbunden ist.

9. Implantierbare Elektrode nach Anspruch 7 oder 8, **dadurch gekennzeichnet, dass** der Kern (9) mit einem sich entlang der Längsachse (L) durchgängig durch den Kern (9) erstreckenden Lumen ausgebildet ist.

## Claims

1. An implantable electrode (1) comprising a sheath (4) extending along a longitudinal axis (L) of the electrode (1), the sheath being made of an electrically insulating material, and comprising at least one connection cable (6), wherein the connection cable (6) is at least partly embedded in the sheath (4) and the sheath (4) is hollow-cylindrical, and comprising at least one contact element (5) for electrically contacting muscle cells or nerve ends, wherein the at least one contact element (5) is electrically conductively connected to the connection cable (6) through the sheath (4),
**characterised in that**
the at least one connection cable (6) is arranged between two electrically insulating thermoplastic polymer films, which are permanently thermally connected with one another, wherein the outer film (3)
- is provided on its inner side with the at least one connection cable (6) and
- is provided on its outer side (3), which is opposite the connection cable (6), with the at least one contact element (5),
wherein, to connect the connection cable (6) to the contact element (5), a plated through-hole (7) in the form of a metallised opening connecting the two sides of the film to one another, is provided in the film.

2. The implantable electrode according to claim 1, **characterised in that** the at least one connection cable (6) or selected connection cables (6) or all of the plurality of connection cables (6) are oriented parallel to the longitudinal axis (L) of the electrode (1).

3. The implantable electrode according to claim 1 or 2, **characterised in that** the at least one contact element (5) extends at least in part around the longitudinal axis (L).

4. The implantable electrode according to any one of claims 1 to 3, **characterised in that** the electrode (1) has a plurality of connection cables (6) embedded at least in part in the sheath (4), wherein the connection cables (6) are arranged spaced apart from one another in a circumferential direction (U) of the electrode (1) extending around the longitudinal axis (L).

5. The implantable electrode according to claim 3 or 4, **characterised in that** the electrode (1) has a plurality of contact elements (5).

6. The implantable electrode according to claim 5, **characterised in that** the contact elements (5) are arranged spaced apart from one another along the longitudinal axis (L).

7. The implantable electrode according to any one of claims 1 to 6, **characterised in that** the electrode (1) has a core (9) against which the sheath (4) rests.

8. The implantable electrode according to claim 7, **characterised in that** the sheath (4) is connected to the core (9) in an integrally bonded manner.

9. The implantable electrode according to claim 7 or 8, **characterised in that** the core (9) is formed with a lumen extending continuously through the core (9) along the longitudinal axis (L).

## Revendications

1. Electrode implantable (1) dotée d'une gaine (4) à base d'un matériau isolant électriquement s'étendant le long de l'axe longitudinal (L) de l'électrode (1), et dotée d'au moins une ligne de connexion (6), où la ligne de connexion (6) est au moins partiellement incorporée dans la gaine (4) et la gaine (4) est façonnée en un cylindre creux, et dotée d'au moins un élément de contact (5) permettant une mise en contact électrique de cellules musculaires ou d'extrémités nerveuses, où l'au moins un élément de contact (5) est relié avec la ligne de connexion (6) de manière conductrice électriquement à travers la gaine (4),
**caractérisée en ce que**
l'au moins une ligne de connexion (6) est disposée entre deux films de polymère thermoplastique électriquement isolants et reliées thermiquement de manière permanente l'un à l'autre, où le film se situant à l'extérieur (3)
- est muni sur sa face intérieure de l'au moins une ligne de connexion (6), et
- est muni sur sa face extérieure (3), la face de ce film située en face de la ligne de connexion (6), de l'au moins un élément de contact (5),
où, pour la connexion de la ligne de connexion (6) avec l'élément de contact (5), un trou de connexion (7) sous la forme d'un orifice métallisé reliant les deux faces du film l'une à l'autre est prévu dans le film.

2. Electrode implantable selon la revendication 1, **caractérisée en ce que** l'au moins une ligne de connexion (6), ou des lignes de connexion (6) sélectionnées, ou toutes parmi les lignes de connexion (6), sont orientées parallèlement par rapport à l'axe longitudinal (L) de l'électrode (1).

3. Electrode implantable selon la revendication 1 ou la revendication 2, **caractérisée en ce que** l'au moins un élément de contact (5) s'étend au moins partiellement autour de l'axe longitudinal (L).

4. Electrode implantable selon l'une des revendications 1 à 3, **caractérisée en ce que** l'électrode (1) présente plusieurs lignes de connexion (6) incorporées au moins partiellement dans la gaine (4), où les lignes de connexion (6) sont disposées espacées les unes par rapport aux autres dans une direction circonférentielle (U) de l'électrode (1) s'étendant autour de l'axe longitudinal (L).

5. Electrode implantable selon la revendication 3 ou la revendication 4, **caractérisée en ce que** l'électrode (1) présente plusieurs éléments de contact (5).

6. Electrode implantable selon la revendication 5, **caractérisée en ce que** les éléments de contact (5) sont disposés espacés les uns des autres le long de l'axe longitudinal (L).

7. Electrode implantable selon l'une des revendications 1 à 6, **caractérisée en ce que** l'électrode (1) présente un cœur (9) auquel la gaine (4) est adjacente.

8. Electrode implantable selon la revendication 7, **caractérisée en ce que** la gaine (4) est reliée avec le cœur (9) par complémentarité des matières.

9. Electrode implantable selon la revendication 7 ou la revendication 8, **caractérisée en ce que** le cœur (9) est conçu avec une lumière s'étendant le long de l'axe longitudinal (L) de part en part à travers le cœur (9).
